# EUROPEAN PATENT SPECIFICATION

(11) **EP 2 685 919 B1**
(45) Date of publication and mention of the grant of the patent: **03.12.2014**
(21) Application number: 12704625.8
(22) Date of filing: 09.02.2012
(51) Int. Cl.: A61B 17/34, A61B 17/00, A61B 17/04, A61M 25/06, A61M 25/00

(54) **TRANSSEPTAL PUNCTURING DEVICE**
TRANSSEPTALE PUNKTIONSVORRICHTUNG
DISPOSITIF DE PONCTION TRANSSEPTALE

(30) Priority: 17.03.2011 US 201113050233
(43) Date of publication of application: 22.01.2014
(73) Proprietor: Medtronic Ablation Frontiers LLC, Minneapolis, MN 55432 (US)
(72) Inventor: BENSCOTER, Mark A., Dellwood, Minnesota 55110 (US); KNUTSEN, Kevin A., Ramsey, Minnesota 55303 (US)
(74) Representative: Hughes, Andrea Michelle
(86) International application number: PCT/US2012/024427
(87) International publication number: WO 2012/125239

(56) References cited:
- WO-A1-92/06733
- WO-A1-2010/125159
- US-A1- 2005 149 097
- US-A1- 2009 182 281
- US-A1- 2011 054 487

## Description

### FIELD

The present disclosure is related to vascular access. More specifically, the disclosure relates to devices and methods for traversing tissue areas including, for example, an inter-atrial septum for transseptal procedures.

### BACKGROUND

Minimally invasive surgical techniques are known for performing medical procedures within all parts of the cardio-vascular system. Exemplary known procedures include the steps of passing a small diameter, highly-flexible catheter through one or more blood vessels and into the heart. When positioned as desired, additional features of the catheter are used, in conjunction with associated equipment, to perform all or a portion of a medical treatment, such as vessel occlusion, tissue biopsy, or tissue ablation, among others. It is often required to create a perforation in the atrial septum to gain access to the left side of the heart during angioplasty, tissue ablation, and other diagnostic and therapeutic procedures to study or treat electrical or morphological abnormalities.

Transseptal puncture devices are used by physicians who perform these specialized cardiology procedures on the left atrium of a heart. Historically in these instances, a needle such as the transseptal needle sold by Medtronic/AVE under the tradename "Brockenbrough®" is used. The needle is made of a stiff metal cannula, and has a sharpened distal tip. The needle is introduced through a guiding sheath in the femoral vein and advanced through the vasculature into the right atrium. From there the needle tip is positioned at the fossa ovalis, the preferred location on the septum for creating a perforation. Once in position, the operator applies force at the proximal end of the needle and uses mechanical energy to advance the needle through the septum and into the left atrium. Once in the left atrium the needle can be attached to an external pressure transducer and the operator can confirm a left atrial pressure before continuing with the procedure. Examples of subsequent steps may include advancing the guiding sheath over the needle and into the left atrium to provide access for other devices to the left heart, or using another device to enlarge the hole made by the needle if a shunt is desired.

In such cardiology procedures, it is also often required to advance a guidewire through to the left atrium. As such, an exchange is performed whereby the operator removes the needle from the guiding sheath and advances the guidewire in its place.

The needles used in this procedure are generally required to be very stiff to enable the desired perforation of the septum wall. The force applied by the needle usually causes the septum to tent, or buckle, before it perforates the tissue. In addition, the amount of force required to perforate the septum varies with each patient. Once the needle makes the perforation, the needle may have significant forward momentum, which can be difficult to control. As such, an unintended consequence of this structure is that the stiff needle can cause damage to the cardiac tissue during advancement of the needle through the vasculature or in the perforation procedure.

Therefore, what is needed is a low-cost, easy to use transseptal puncturing device and methods of use thereof that address the drawbacks of current devices and methods.

WO 92/06733 A1 is regarded as being the prior art closest to the subject-matter of claim 1 and discloses a transseptal puncturing device comprising an elongate tubular body having a central lumen having a proximal opening at the proximal end and a distal opening at the distal end and further having an offset lumen having a longitudinal length extending partially within the length of the tubular body said internal lumen being coextensive at least in part with the central lumen and having a first opening terminating within the length of the tubular body, wherein the first opening is in fluid communication with the central lumen. This device further comprises a handle coupled to the distal end of the tubular body. The handle having a delivery actuator and the needle being preloaded within the offset lumen and coupled to the delivery actuator.

US2011/054487 teaches a further transeptal puncturing assembly.

### SUMMARY

The disclosure provides a device for creating a perforation in material of a patient, including in particular, material that comprises the interatrial septum. Among other things, the device facilitates the simplification of procedures requiring transseptal access by eliminating the need for exchange of a needle for a guidewire and improving maneuverability of the device. The disclosure seeks to provide a device that is associated with a decreased risk of unintentional injury to other areas of the heart.

In accordance with the foregoing, there is provided a device for creating a puncture in material within a patient. The device comprises an elongate member having a handle at the proximal region. The device includes a dual lumen structure comprising a central lumen and an offset lumen. The central lumen has a proximal opening at a proximal end and a distal opening at a distal end of the elongate member. The offset lumen extends partially through a longitudinal length of the tubular body and is coextensive at least in part with the central lumen. A first opening is provided at the distal end of the offset lumen with the first opening being in fluid communication with the central lumen to thereby terminate the offset lumen within the longitudinal length of the tubular body.

According to aspects of this disclosure, the device integrates a puncturing needle into the dual lumen elongate tube. The extension of the needle beyond a distal endface of the elongate tube may be limited by an actuator coupled in the handle of the device.

In an alternative aspect of this disclosure, the device may comprise a vacuum port for coupling the proximal opening to a vacuum source. The central lumen may be configured at the proximal region for fluid communication with the vacuum source and configured at the distal region for fluid communication with an environment about the distal region. The vacuum source applies a suction force through the central lumen to the distal endface and thereby pulls tissue adjacent the distal endface proximally in relation to the device.

A method for accessing the heart of a patient is also described; including positioning a transseptal device proximate to a septal wall, the transseptal device including a central lumen and an offset lumen and the transseptal device further including a needle movably disposed within the offset lumen.

In an example, the method comprises the steps of inserting the transseptal device into a vessel of a patient towards a right atrium of the heart. The needle and steering element are located simultaneously in the transseptal device. In some examples, there is the step of threading a steering element through the transseptal device. In other examples, the steering element is preloaded in the transseptal device. Next, the transseptal device is moved with the needle disposed therein to the puncture site at the right atrium such as the inter-atrial septum wall. The needle is then advanced from the offset lumen into the central lumen and out through the distal endface opening of the transseptal device. Next, the septum wall is punctured by further advancing the needle through to the left atrium of the heart. The method may include engaging at least a portion of the septal wall with the transseptal device prior to creation of the opening, where the engagement includes the application of a suction force through at least one of the lumens.

The foregoing summary information is intended to merely illustrate some of the aspects and features of the present disclosure and is not meant to limit the scope in any way. In fact, upon review of the foregoing and the following described and depicted embodiments, one of skill in the art will surely recognize insubstantial modifications or extensions of the disclosure each of which is expressly intended to be covered hereby.

### BRIEF DESCRIPTION OF THE DRAWINGS

The following drawings are illustrative of particular embodiments of the present disclosure and therefore do not limit the scope of the disclosure. The drawings (not to scale) are intended for use in conjunction with the explanations in the following detailed description, wherein similar elements are designated by identical reference numerals. Moreover, the specific location of the various features is merely exemplary unless noted otherwise.
FIG. 1 depicts an exemplary transseptal device of the present disclosure as it would be deployed and used for performing a transseptal puncturing procedure.
FIG. 2 illustrates an exemplary embodiment of a transseptal device.
FIG. 3 illustrates a cross-sectional side view of the exemplary transseptal device of FIG. 2.
FIGS. 4-6 depict sequentially an exemplary method for puncturing a hole in a patient's cardiac tissue.

### DETAILED DESCRIPTION

The following detailed description is merely exemplary in nature and is not intended to limit the disclosure or the application and uses of the disclosure. Furthermore, there is no intention to be bound by any expressed or implied theory presented in the preceding technical field, background, brief summary or the following detailed description.

The devices and methods of this disclosure may be used within body lumens, chambers or cavities of a patient for diagnostic or therapeutic purposes such as, for example, in procedures where access to internal body regions is to be achieved through the vascular system or other vessels without complex invasive surgical procedures. For example, the devices and methods described may, for example, be used to create a puncture in the fossa ovalis region of the atrial septum during the diagnosis or treatment of heart conditions. While the exemplary implementations described herein are presented as they may be used in conjunction with diagnosis or treatment of heart conditions, they may also have application in the diagnosis or treatment of conditions in other regions or organs of the body such as the prostate, liver, brain, and gall bladder.

Features or configurations of the devices or steps of the present disclosure enable simplification and efficiency in the creation of a transseptal puncture. The device integrates a puncturing needle into a dual-lumen elongate tube. It is to be understood that references to puncture or puncturing a material, such as tissue, in relation to the present disclosure includes piercing, perforating, cutting, ablating, coagulating, removing material and creating a hole.

The objects, such as needles, used in conjunction with the devices and methods of the disclosure to create a puncture are generally desired to be relatively sharp. These objects may come into contact with other surfaces, including the wall of an associated elongate tube such as a dilator, to cause skiving of particles from the elongate tube. Protection against contact between the adjacent objects helps to reduce the production of skived particles. Several examples of the devices and methods of this disclosure contribute to a reduction of the occurrences of skiving.

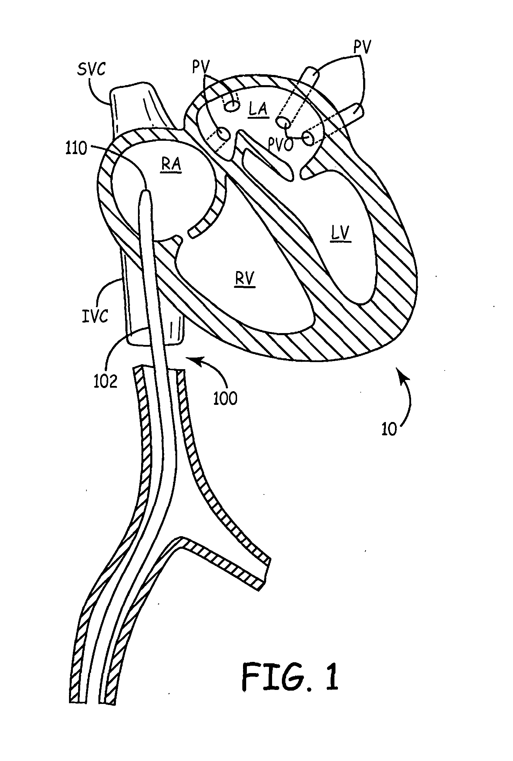

FIG. 1 depicts an exemplary transseptal device of the present disclosure as it would be deployed and used for performing a transseptal puncturing procedure. A transseptal device 100 is illustrated being inserted within a heart 10. Generally, the devices described herein are sized and configured to be easily maneuvered and extended into the right atrium and thereafter into the left atrium. As a point of reference, the heart 10 includes a right atrium (RA), a left atrium (LA), a right ventricle (RV) and a left ventricle (LV). An inferior vena cava (IVC) and a superior vena cava (SVC) lead into the RA. The RA is separated from the LA by an interarterial septum 14 typically having a latent Fossa Ovalis valve formed therethrough. This latent valve provides an appropriate site to introduce a medical instrument such as a mapping and/or ablation catheter to provide diagnosis and therapy to portions of the left atrium LA. Finally, four pulmonary veins (PV) extend from the left atrium LA. Each of the pulmonary veins PV forms an ostium (PVO) in the left atrium LA wall. The heart 10 may also be formed such that a separate ostium PVO is not formed for each individual pulmonary vein PV. In other words, a single pulmonary vein ostium PVO may be formed for two pulmonary veins PV. For example, a single pulmonary vein ostium PVO may be formed for both the left inferior pulmonary vein PV and the left superior pulmonary vein PV, with the two pulmonary veins PV bifurcating from the single ostium PVO.

Alternatively, the introduction of a distal portion 102 of the transseptal device 100 into the right atrium RA is also suggested by passage of the distal portion into the right atrium RA through the superior vena cava SVC.

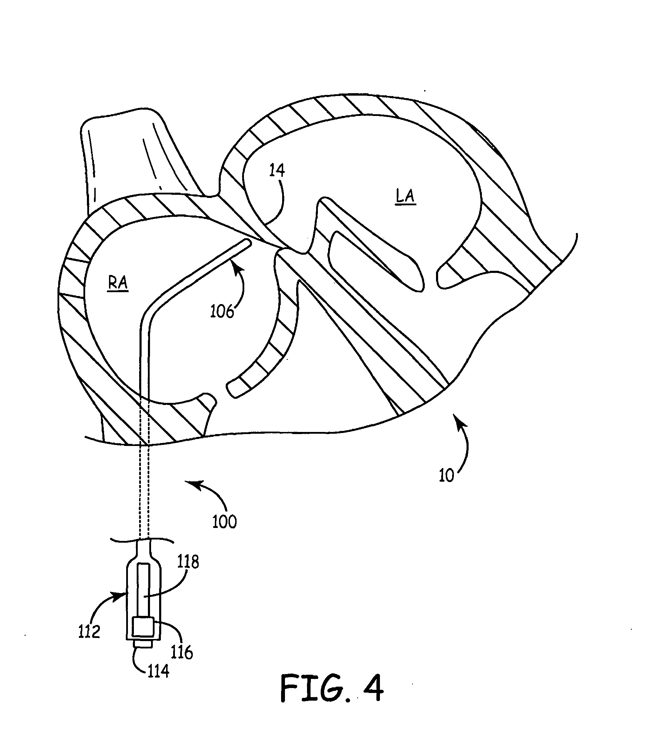

As shown in FIG. 1, access into the left atrium LA begins by directing distal portion 102 of the transseptal device 100 through the inferior vena cava IVC and into the right atrium RA. The transseptal device 100 is then manipulated to create a puncture in the interarterial septum 14 preferably located at the site of a latent Fossa Ovalis valve (as suggested in FIGS. 4-6) and into the left atrium LA. In practice, a clinician may optionally confirm (or reconfirm) that the distal endface 110 of the transseptal device 100 is aligned or oriented appropriately prior to punturing the septal wall. The optional step may involve fluoroscopy with or without dispensing marker material in or near the region of the septal wall.

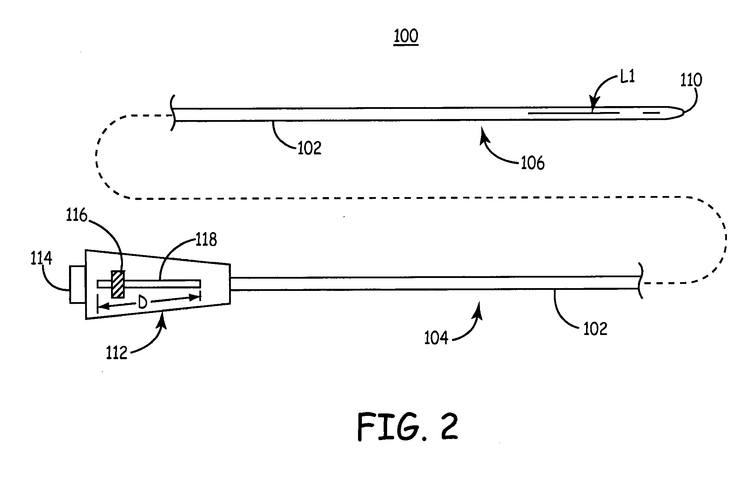

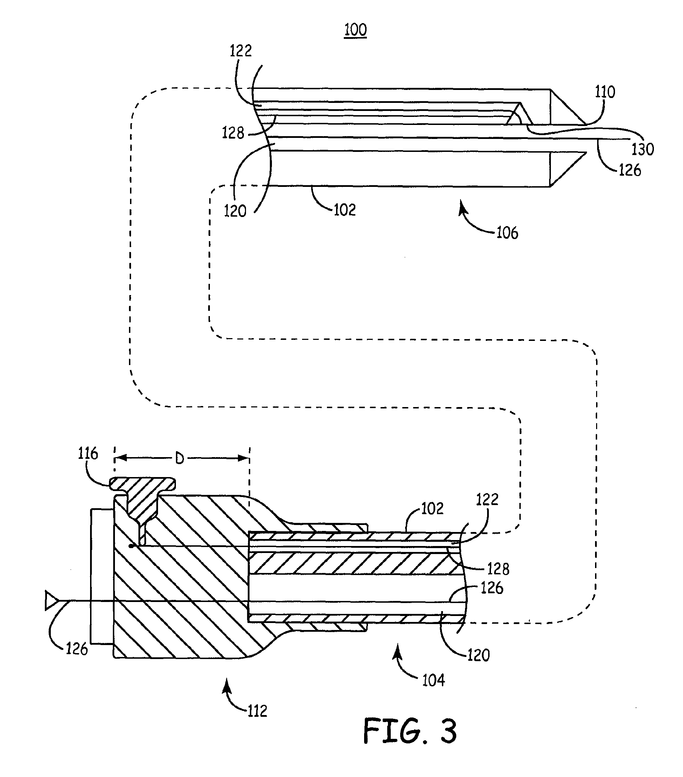

FIG. 2 illustrates an exemplary embodiment of a transseptal device. Transseptal device 100 includes an elongate tube 102 having a distal portion 106 that has a pre-formed tapered profile narrowing towards distal endface 110 to help in properly positioning the distal portion. The elongate tube 102 also includes a proximal portion 104 and includes, at least, two lumens (FIG. 3). As described in greater detail below, the transseptal device 100 also includes a handle 112 coupled to the proximal portion 104 of elongate tube 102.

Regarding fabrication techniques for elongate tube 102, the proximal portion 104 and the distal portion 106 may be integrally formed from a biocompatible material having requisite strength and flexibility for deployment within heart 10. Appropriate materials can be comprised of a biocompatible polymer as well as other known biocompatible materials including polyamides. The elongate tube 102 can be injection molded from a material having a relatively high modulus of elasticity, yet being sufficiently elastic and not prone to brittle fracture, for example 75D durometer polyurethane or high density polyethylene or polyamide. Alternately, one or both of the proximal portion 104 and the distal portion 106 can be insert-molded or formed by molding or an extrusion process. According to some embodiments, portions can be wholly or partially formed from a metal having suitable elastic and elastomeric properties, examples of which include, but are not limited to, titanium alloys, Ni-Ti super-elastic alloys and stainless steel and the like. Other suitable materials known in the art can also be used. In another embodiment, the proximal portion 104 and the distal portion 106 can be individually fabricated.

The proximal portion 104 and the distal portion 106 are preferably flexible, so as to facilitate desired articulation during use. In general terms, however, the elongate tube 102 defines a longitudinal axis L1. It should be recognized that in one position, the longitudinal axis L1 extends linearly from the proximal portion 104 to the distal portion 106. Upon deployment, it may be that the distal portion 106 is forced to an arcuate, curved or curvilinear orientation. With this in mind, the distal portion 106 may have a curvilinear shape that is pre-formed in a plane transverse to the longitudinal axis L1. To this end, at least part of the distal portion 106 preferably extends in a generally arcuate fashion. In other examples, various configurations of the distal portion of the elongate tube 102 including linear, curvilinear or complex distal portions may be employed according to the present disclosure.

Regardless of the exact shape, the distal portion 106 preferably orients the distal endface 110 toward the atrial septum as described in conjunction with FIG. 1. As such, the distal portion 106 facilitates the positioning of the distal endface 110 adjacent to the atrial septum. With this particular application, the distal portion 106 is preformed to assume an arc when deployed with the arc having a diameter suitable for insertion through the vasculature as is known in the art. The pre-shaped distal portion 106 may be formed in a variety of ways, such as by incorporating a preformed section of super elastic, shape memory material, such as Nitinol, with a loop configuration. To facilitate guiding of the distal portion 106 into a heart (FIG. 1), the elongate tube 102 may include a steering element (FIG. 3) internally disposed within the elongate tube 102. In an extended position, the steering element would extend through the distal portion 106, so as to render the pre-shaped portion straight. Upon retraction of the steering element, the distal portion 106 would take on the preformed shape.

Alternatively, a delivery catheter (not shown) may be employed for delivering the elongate tube 102. The delivery catheter will slidably receive the elongate tube 102. Prior to deployment, the distal portion 106 would be retracted within the delivery catheter, rendering the pre-shaped portion straight. Upon deployment from the delivery catheter, the distal portion 106 would take on the preformed shape. Any other approaches known in the art for providing and delivering preformed devices are similarly acceptable.

A portion of the distal portion 106 may be tapered or otherwise define a gradually narrowing profile, in relation to the proximal portion 104, towards the distal endface 110 of the elongate tube 102. Tapering the distal portion 106 will facilitate maneuvering of the elongate tube 102 through the tortuous vasculature to a targeted puncture site.

The elongate tube 102 may be coupled at its proximal end 104 through, for example, a positive locking mechanism (such as a snap fit) to handle 112 used to manipulate and position the transseptal device 100. The exemplary handle 112 may be sized to be grasped by a user and includes a port 114. Port 114 includes a channel that is in fluid communication with a channel of the handle 112 such that both channels are in fluid communication with one or both lumens 120, 122. The handle 112 will typically be formed from inert plastics or other materials that are suitable to medical applications. Specifically, the handle may be formed from any suitable material such as plastics, polymers, non-ferrous metals such as gold or copper, or a mixture thereof.

An actuator 116 which is connected to handle 112 may be used to advance a needle (FIG. 3) distally through manipulation of the actuator 116. The actuator 116 is independently moveable, in relation to handle 112, within a channel 118 that has a predetermined distance (D). For example, the distance may range in length between 0.1 mm to 4 mm. The actuator 116 is coupled to the needle such that movement of the actuator 116 in the distal direction imparts lateral movement to deploy the needle from the elongate tube 102 and movement of the actuator 116 in the proximal direction retracts the needle to conceal it within the elongate tube 102. The coupling between the actuator 116 and the needle may be through a mechanical connection such as a setscrew, welding, a snap-fit connection or any other suitable coupling techniques. The predetermined distance D of channel 118 is chosen so that when the needle is fully advanced within the elongated tube 102, the needle tip will traverse the atrial septum the desired distance, but not so far as to contact the opposite wall of the left atrium, or other adjacent tissue. Hence, the needle 128 will extend from the distal endface 110 by a length approximately corresponding to the length by which the actuator 116 is moved along the channel 118 (less the distance to which the needle 128 is retracted within elongate tube 102).

In other embodiments, actuator 116 may be formed as a wheel that is rotated about an axis to impart lateral movement of the needle 128. In such an embodiment, the rotational distance would translate into the lateral length over which the needle 128 is extended. In either configuration, the actuator 116 will serve to indicate to a user the length to which the needle has been extended. In addition to this benefit, the assembly of the transseptal device 100 having handle 112 and incorporating a puncturing needle facilitates a one-handed manipulation of the device 100 of the present disclosure. This aspect facilitates ease of use and simplifies the puncturing procedure.

FIG. 3 illustrates a cross-sectional side view of the exemplary transseptal device 100 of FIG. 2. The elongate tube 102 includes a central lumen or channel 120. Elongate tube 102 also includes an offset lumen 122 defined therein extending through at least a portion of the tube 102. The offset lumen 122 may at least be coaxially aligned with the central lumen 120. In the illustrative embodiment, the offset lumen 122 is disposed entirely within the elongate tube 102; having a length that is shorter in relation to the length of the elongate tube 102. The offset lumen 122 is configured to terminate in an internal converging surface partway through the length of the distal portion 106 of elongate tube 102. As such, offset lumen 122 includes a first opening 130 that is in fluid communication with the central lumen 120.

As is further illustrated in FIG. 3, a steering element 126 may be slidably received through central lumen 120. Examples of the steering element 126 may include a guidewire, stylet, catheter or a tubular member that is suitable for introduction through the proximal opening of central lumen 120 and slidably moveable through the central lumen 120 to the distal endface opening 110.

Central lumen 120 may also be used for injection of a fluid medium that may be delivered through distal endface 110 to a surrounding environment. As is known in the art, such fluid delivery may include isotonic saline solution to form so-called virtual electrode regions, marker dye or other material that is advantageously dispensed in, on or near a cardiac tissue location.

A needle 128 may be preloaded, or positioned, in the offset lumen 122. The needle 128 can take a number of configurations. One such needle 128 is a transseptal needle such as the Brockenbrough® needle discussed above. Another example is a hollow, substantially cylindrical needle element that may be formed from hypotubes. In addition to hypotubes, needle elements may be formed from a reinforced plastic, a pultruded material, a glass or carbon reinforced plastic, reinforced ABS, or high impact ABS or any other plastic material capable of maintaining a sharp tip and transmitting the force required for a given puncturing application. Other configurations include solid access or puncture devices, and hollow tube elements having side access ports at the distal end portions which allow fluid to exit at least one side of the needle element. Hollow needle elements also permit the monitoring of blood pressure within the right and left atria. As is known, the blood pressure within the left and right atrium is substantially different, and this fact can be used as evidence to confirm that a successful puncture of the atrial septum has been performed. However, the disclosure is not limited to any particular type of needle and any suitable element that can function to puncture material in a patient may be used.

With continued reference to FIG. 3, the distal portion of needle 128 may include a pre-formed curve that is complimentary to the distal portion 106 such that the needle 128 will conform to the curvature of the associated elongated tube 102. The needle 128 is coupled to actuator 116 on the handle 112. With that in mind, the needle 128 is moveable within the offset lumen 122 with the movement being imparted through actuation of actuator 116. For example, the needle 128 is entirely disposed within the elongate tube 102 in a first configuration and partially withdrawn from the distal endface 110 of the elongate tube 102 in a second configuration. In that example, the actuator 116 is located toward the proximal-most section of channel 118 in the first configuration and toward the distal-most section of channel 118 in the second configuration.

Central lumen 120 may be relatively constant in cross-sectional area over the entire length of tube 102 or, in other embodiments over, a majority of the length of tube 102. When formed with varying cross-sectional areas, the distal portion of the central lumen 120 terminating at the distal endface 110 may be configured with an area that will permit both the steering element 126 and the needle 128 to contemporaneously reside therein and be withdrawn through the distal endface 110 opening. The central lumen 120 may be centralized within the elongate tube 102 parallel to longitudinal axis L1 (shown in FIG. 2), or may be arranged in an eccentric configuration with respect to the tube 102 and/or other lumens (e.g., offset lumen 122).

In order to be suitable for its intended purpose, the needle 128 typically includes a sharp distal end to enable the puncturing that will make easier entry into tissue or other material on which the needle 128 is to be used. By preloading the needle 128 in the offset lumen 122, movement of the sharp tip of needle 128 is limited to the extension imparted by actuator 116. The presence of both the central lumen 120 and the offset lumen 122 permits the contemporaneous disposition of both the needle 128 and the steering element 126 within the elongate tube 102. This eliminates the requirement for the exchange of needle 128 with steering element 126 as is the case with the conventional devices that permit only one or the other of either steering element 126 or needle 128 to be in the device 100 at one time. Moreover, preloading of needle 128 within offset lumen 122 and the limiting of the movement within the lumen 122 facilitates the reduction or elimination of skiving of particles from the elongate tube 102.

In an embodiment, a vacuum source (not shown) may be connected to elongate tube 102 via the port 114 on handle 112. The port 114 is in fluid communication with the central lumen 120. As such, when a vacuum source is connected to the port 114 at least a partial vacuum is established within the central lumen 120 of the elongate tube 102. The application of a vacuum or suction to the targeted tissue by the device 100 aids in both tensioning the tissue to allow easier penetration by needle 128 as well as preventing the pierced tissue from "tenting" or extending outward from the opposite side of the tissue wall 120 (into the left atrium, for example), thereby decreasing the likelihood of any complications or unintended trauma to surrounding tissue structures from the tissue penetration. Once the tissue has been crossed or otherwise pierced, the vacuum/suction application may be discontinued.

FIGS. 4-6 more particularly illustrate sequentially an exemplary method for puncturing a hole in a patient's cardiac tissue. At the start of the procedure, an incision is made in the skin adjacent to the femoral vein. The transseptal device 100 is inserted through the incision into the femoral vein and advanced, for example with the aid of fluoroscopy, through the vasculature into the right atrium RA and positioned so that the distal portion 106 is adjacent the location of the procedure. In the example shown, the location is the fossa ovalis, the preferred location on the septum 14 for creating a perforation, but any location could be used.

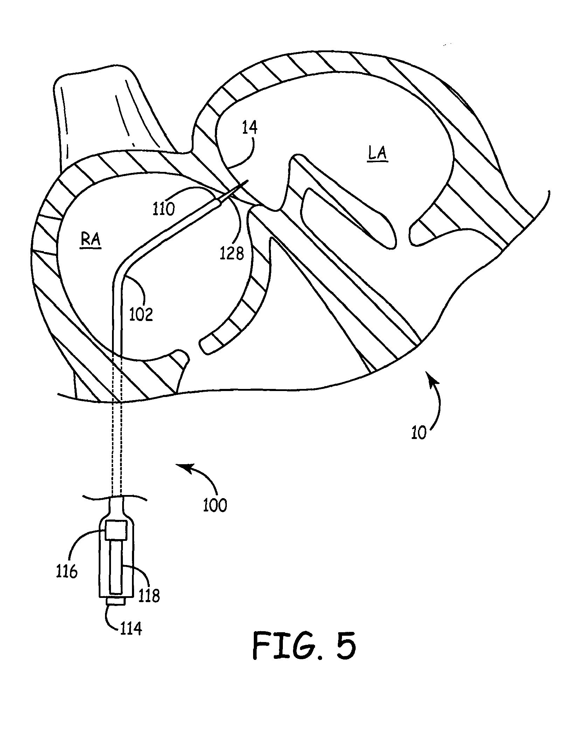

Referring to FIG. 5, the operator may then advance the actuator 116 and the motion of the actuator 116 is translated to lateral movement of the needle 128. As previously described, the actuator 116 movement causes the needle 128 to extend into the central lumen 120 and exit the distal endface 110 opening of the elongate tube 102. The needle 128 creates a puncture in the septum wall as it is advanced past the distal endface 110. The advancing step involves slidably moving the actuator 116 in a distal direction along the length of channel 118. The forward movement causes needle 128 to sequentially prick and then puncture the septum wall. The actuator 116 may be configured to limit or otherwise control passage of a portion of the needle 128 through the septum wall to prevent overextension or positioning into a region opposite the punctured wall, which could result in injury. In other words, the extension of needle 128 is limited to the length of channel 118 which thereby facilitates the reduction of unintended over-extension and subsequent undesired perforation of other cardiac tissue.

In alternative embodiments, a vacuum supply source connected to the port 114 of handle 112 may be energized prior to advancing the needle 128. Energizing the vacuum supply source will evacuate air from the central lumen 120 of elongate tube 102 which is in contact with the cardiac tissue adjacent the fossa ovalis. The suction force releasably secures the procedure site of the fossa ovalis. The needle 128 will create a puncture in the septum wall while the septum is held fast against the elongate tube 102 by the vacuum created by the vacuum supply source. The action of the vacuum holding fast the septum during creation of the puncture may aid with minimizing deflection (or tenting) of the tissue in proximity to the puncture. After the hole is created in the septum wall 14, and with needle 128 in the fully advanced position, the vacuum system is deactivated.

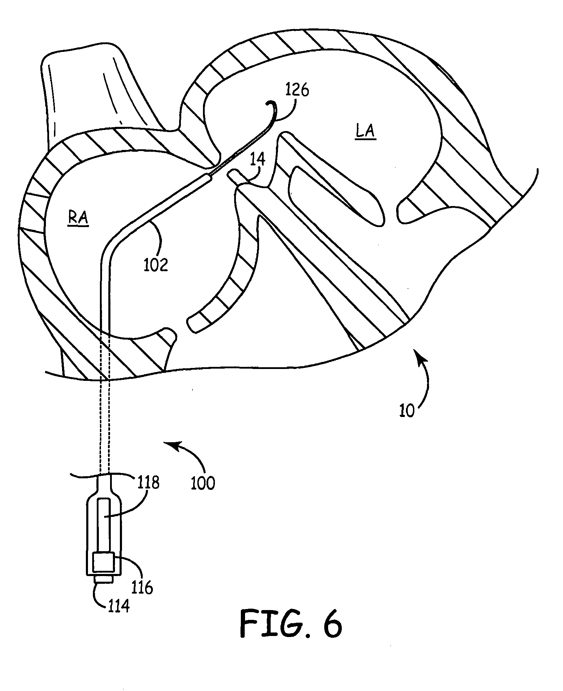

Turning next to FIG. 6, subsequent to creation of the hole, the needle 128 can be retracted to leave a hole in the septum wall for access of other tools and devices. For example, a steering element 126 may be introduced into the left atrium (LA) through the puncture hole that was created in the septum wall to maintain the position of the distal portion 106 of the elongate tube 102 with respect to the puncture site. The steering element 126 may be pre-loaded in the central lumen 120 prior to the start of the procedure or introduced subsequently. If subsequently introduced, the steering element 126 is threaded from the proximal opening of elongate tube 102 through the central lumen 120 towards the distal endface 110 opening. In either event, the transseptal device 100 of the present disclosure facilitates the simultaneous placement of the steering element 126 and needle 128 within the elongate tube 102.

While the present disclosure has been described in terms of certain exemplary preferred embodiments, it will be readily understood and appreciated by one of ordinary skill in the art that it is not so limited and that many additions, deletions and modifications to the preferred embodiments may be made within the scope of the disclosure as hereinafter claimed. It should also be understood that the described features, configurations or steps are presently-contemplated examples, but other examples with other features, configurations or steps or combinations thereof may also achieve one or more of the benefits of the present disclosure. Accordingly, the scope of the disclosure is limited only by the scope of the appended claims.

## Claims

1. A transseptal puncturing device, comprising
a dual lumen elongate tubular body (102) having a proximal end (104) and a distal end (106); and comprising
a central lumen (120) within said tubular body having a proximal opening at the proximal end and a distal opening at the distal end; and
an offset lumen (122) having a longitudinal length extending partially within the length of the tubular body said offset lumen being coextensive at least in part with the central lumen and having a first opening (130) terminating within the length of the tubular body, wherein the first opening is in fluid communication with the central lumen;
a handle (112) coupled to the distal end of the tubular body; said handle having a delivery actuator (116); and
a needle (128) preloaded within the offset lumen and coupled to the delivery actuator;
wherein the needle is moveable from a retracted position to an extended position in response to actuation of the delivery actuator.

2. The transseptal puncturing device of claim 1, wherein the delivery actuator (116) is slidably moveable in an axial direction between a first position and a second position to trigger movement of the needle (128) between the retracted position and the extended position.

3. The transseptal puncturing device of claim 1, wherein the delivery actuator (116) is configured to permit axial movement of the needle (128) to a distance ranging between 0.5 mm to 2.5 mm.

4. The transseptal puncturing device of claim 1, wherein the delivery actuator (116) is rotatably moveable to trigger axial movement of the needle (128) between the retracted position and the extended position.

5. The transseptal puncturing device of claim 1, further comprising a guidewire (126) slidably disposed within the central lumen.

6. The transseptal puncturing device of claim 1, wherein a distal tip of the needle (128) is configured for piercing a body part.

7. The transseptal puncturing device of claim 1, wherein the distal tip of the needle (128) is disposed fully within the offset lumen (122) in the retracted position and a portion of the needle is advanced through the first opening (130) into the central lumen (120) and withdrawn from the tubular body (102) through the distal opening in the extended position.

8. The transseptal puncturing device of claim 1, wherein a first portion of the central lumen (120) defines a first cross-sectional diameter and a second portion of the central lumen defines a second cross-sectional diameter.

9. The transseptal puncturing device of claim 8, wherein the second portion of the central lumen (120) is configured to contemporaneously permit the needle (128) and a second medical device to be disposed therein.

10. The transseptal puncturing device of claim 9, wherein the second medical device is a guidewire.

11. The transseptal puncturing device of claim 1, further comprising an injection port (114) disposed at the proximal end, wherein the central lumen is in fluid communication with the injection port, said injection port being configured for introduction of contrast medium into the central lumen.

12. The transseptal puncturing device of claim 1 further comprising a suction fitting coupled to the proximal opening of the central lumen (120), wherein the suction fitting is adapted for connection to a vacuum source for application of suction at the distal opening through the central lumen.

13. The transseptal puncturing device of claim 1, wherein at least a distal portion of the tubular body (102) is curved.

14. The transseptal puncturing device of claim 1, wherein the needle (128) is made from material having shape memory properties.

15. The transseptal puncturing device of claim 1, wherein a distal portion of the tubular body is tapered toward the distal end.

## Patentansprüche

1. Transseptale Punktionsvorrichtung, mit einem Doppellumen-Verlängerungsrohrkörper (102) mit einem proximalen Ende (104) und einem distalen Ende (106); und mit
einem zentralen Lumen (120) innerhalb des Rohrkörpers mit einer proximalen Öffnung an dem proximalen Ende und einer distalen Öffnung an dem distalen Ende; und
einem versetzt angeordneten Lumen (122) mit einer Länge in Längsrichtung, die sich teilweise innerhalb der Länge des Rohrkörpers erstreckt, wobei das versetzt angeordnete Lumen koextensiv zumindest teilweise mit dem zentralen Lumen ist und eine erste Öffnung (130) aufweist, die innerhalb der Länge des Rohrkörpers endet, wobei die erste Öffnung in Fluidverbindung mit dem zentralen Lumen steht;
einem Handgriff (112), der mit dem distalen Ende des Rohrkörpers gekoppelt ist, wobei der Griff einen Abgabeaktor (116) aufweist;
einer Nadel (128), die innerhalb des versetzt angeordneten Lumens vorgespannt und mit dem Abgabeaktor (116) gekoppelt ist;
wobei die Nadel aus einer eingezogenen Position in eine ausgefahrene Position in Reaktion auf die Betätigung des Abgabeaktors bewegbar ist.

2. Transseptale Punktionsvorrichtung nach Anspruch 1, wobei der Abgabeaktor (116) in axialer Richtung zwischen einer ersten Position und einer zweiten Position gleitend beweglich ausgebildet ist, um eine Bewegung der Nadel (128) zwischen der eingezogenen Position und der ausgefahrenen Position auszulösen.

3. Transseptale Punktionsvorrichtung nach Anspruch 1, wobei der Abgabeaktor (116) ausgebildet ist, um eine axiale Bewegung der Nadel (128) bis zu einem Abstand im Bereich von 0,5 mm bis 2,5 mm zu ermöglichen.

4. Transseptale Punktionsvorrichtung nach Anspruch 1, wobei der Abgabeaktor (116) drehbeweglich ist, um eine axiale Bewegung der Nadel (128) zwischen der eingezogenen Position und der ausgefahrenen Position auszulösen.

5. Transseptale Punktionsvorrichtung nach Anspruch 1, mit einem Führungsdraht (126), der verschiebbar in dem zentralen Lumen angeordnet ist.

6. Transseptale Punktionsvorrichtung nach Anspruch 1, wobei eine distale Spitze der Nadel (128) zum Durchstechen eines Körperteils ausgebildet ist.

7. Transseptale Punktionsvorrichtung nach Anspruch 1, wobei die distale Spitze der Nadel (128) vollständig in dem versetzt angeordneten Lumen (122) in der eingezogenen Position angeordnet ist und ein Abschnitt der Nadel durch die erste Öffnung (130) in das zentrale Lumen (120) vorgeschoben und von dem Rohrkörper (102) durch die distale Öffnung in der ausgefahrenen Position zurückgezogen ist.

8. Transseptale Punktionsvorrichtung nach Anspruch 1, wobei ein erster Abschnitt des zentralen Lumens (120) einen ersten Querschnittsdurchmesser und ein zweiter Abschnitt des zentralen Lumens einen zweiten Querschnittsdurchmesser definiert.

9. Transseptale Punktionsvorrichtung nach Anspruch 8, wobei der zweite Abschnitt des zentralen Lumens ausgebildet ist, um gleichzeitig ein Anordnen der Nadel (128) und einer zweiten medizinische Vorrichtung darin zu ermöglichen.

10. Transseptale Punktionsvorrichtung nach Anspruch 9, wobei die zweite medizinische Vorrichtung ein Führungsdraht ist.

11. Transseptale Punktionsvorrichtung nach Anspruch 1, weiter umfassend eine Injektionsöffnung (114) an dem proximalen Ende, wobei das zentrale Lumen in Fluidverbindung mit der Injektionsöffnung steht, wobei die Injektionsöffnung zum Einführen von Kontrastmittel in das zentrale Lumen ausgebildet ist.

12. Transseptale Punktionsvorrichtung nach Anspruch 1, ferner mit einem Saugstutzen, gekoppelt mit der proximalen Öffnung des zentralen Lumens (120), wobei der Saugstutzen zum Verbinden mit einer Vakuumquelle zum Anlegen von einem Sog an der distalen Öffnung durch das zentrale Lumen ausgebildet ist.

13. Transseptale Punktionsvorrichtung nach Anspruch 1, wobei mindestens ein distaler Abschnitt des Rohrkörpers (102) gekrümmt ausgebildet ist.

14. Transseptale Punktionsvorrichtung nach Anspruch 1, wobei die Nadel (128) aus einem Material mit Formgedächtniseigenschaften gefertigt ist.

15. Transseptale Punktionsvorrichtung nach Anspruch 1, wobei ein distaler Abschnitt des Rohrkörpers sich in Richtung des distalen Endes verjüngend ausgebildet ist.

## Revendications

1. Dispositif de ponction transseptale comportant :
un corps tubulaire allongé à double lumière (102) ayant une extrémité proximale (104) et une extrémité distale (106), et comportant
une lumière centrale (120) à l'intérieur dudit corps tubulaire ayant une ouverture proximale au niveau de l'extrémité proximale et une ouverture distale au niveau de l'extrémité distale ; et
une lumière de compensation (122) ayant une longueur longitudinale qui s'étend partiellement à l'intérieur de la longueur du corps tubulaire, ladite lumière de compensation étant coextensive au moins en partie avec la lumière centrale et ayant une première ouverture (130) se terminant à l'intérieur de la longueur du corps tubulaire, dans lequel la première ouverture est en communication de fluide avec la lumière centrale ;
une poignée (112) couplée à l'extrémité distale du corps tubulaire ; ladite poignée ayant un actionneur de délivrance (116) ; et
une aiguille (128) préchargée à l'intérieur de la lumière de compensation et couplée à l'actionneur de délivrance ;
dans lequel l'aiguille est mobile à partir d'une position rétractée jusqu'à une position étendue en réponse à l'actionnement de l'actionneur de délivrance.

2. Dispositif de ponction transseptale selon la revendication 1, dans lequel l'actionneur de délivrance (116) est mobile par coulissement dans une direction axiale entre une première position et une seconde position pour déclencher un mouvement de l'aiguille (128) entre la position rétractée et la position étendue.

3. Dispositif de ponction transseptale selon la revendication 1, dans lequel l'actionneur de délivrance (116) est configuré pour permettre un mouvement axial de l'aiguille (128) jusqu'à une distance comprise entre 0,5 mm et 2,5 mm.

4. Dispositif de ponction transseptale selon la revendication 1, dans lequel l'actionneur de délivrance (116) est mobile par rotation pour déclencher un mouvement axial de l'aiguille (128) entre la position rétractée et la position étendue.

5. Dispositif de ponction transseptale selon la revendication 1, comportant en outre un fil de guidage (126) disposé par coulissement à l'intérieur de la lumière centrale.

6. Dispositif de ponction transseptale selon la revendication 1, dans lequel une extrémité distale de l'aiguille (128) est configurée pour percer une partie du corps.

7. Dispositif de ponction transseptale selon la revendication 1, dans lequel l'extrémité distale de l'aiguille (128) est disposée complètement à l'intérieur de la lumière de compensation (122) dans la position rétractée et une partie de l'aiguille avance à travers la première ouverture (130) dans la lumière centrale (100) et est retirée du corps tubulaire (102) à travers l'ouverture distale dans la position étendue.

8. Dispositif de ponction transseptale selon la revendication 1, dans lequel une première partie de la lumière centrale (120) définit un premier diamètre en coupe transversale et une seconde partie de la lumière centrale définit un second diamètre en coupe transversale.

9. Dispositif de ponction transseptale selon la revendication 8, dans lequel la seconde partie de la lumière centrale (120) est configurée pour permettre simultanément à l'aiguille (128) et à un second dispositif médical d'être disposés dans celle-ci.

10. Dispositif de ponction transseptale selon la revendication 9, dans lequel le second dispositif médical est un fil de guidage.

11. Dispositif de ponction transseptale selon la revendication 1, comportant en outre un orifice d'injection (114) disposé au niveau de l'extrémité proximale, dans lequel la lumière centrale est en communication fluide avec l'orifice d'injection, ledit orifice d'injection étant configuré pour l'introduction d'un support de contraste dans la lumière centrale.

12. Dispositif de ponction transseptale selon la revendication 1, comportant en outre un raccord d'aspiration couplé à l'ouverture proximale de la lumière centrale (120), dans lequel le raccord d'aspiration est adapté en vue d'une connexion à une source sous vide en vue de l'application d'une aspiration au niveau de l'ouverture distale à travers la lumière centrale.

13. Dispositif de ponction transseptale selon la revendication 1, dans lequel au moins une partie distale du corps tubulaire (102) est incurvée.

14. Dispositif de ponction transseptale selon la revendication 1, dans lequel l'aiguille (128) est constituée d'un matériau ayant des propriétés de mémoire de forme.

15. Dispositif de ponction transseptale selon la revendication 1, dans lequel une partie distale du corps tubulaire est unique en direction de l'extrémité distale.
